# EUROPEAN PATENT SPECIFICATION

(11) **EP 0 436 474 B1**
(45) Date of publication and mention of the grant of the patent: **20.07.1994**
(21) Application number: 90830507.1
(22) Date of filing: 08.11.1990
(51) Int. Cl.: C07C 49/747, C07C 49/753, C07H 15/252, A61K 31/70

(54) **New fluoro-naphtacenediones, their glycosilated derivatives and methods of making them**
Fluornaphthacendione, deren glycosilierte Abkömmlinge und Verfahren zu deren Herstellung
Fluoro-naphtacènediones, leurs dérivés glycosilés et procédé pour leur fabrication

(30) Priority: 13.11.1989 IT 956489; 20.02.1990 IT 932190; 30.03.1990 IT 935790
(43) Date of publication of application: 10.07.1991
(73) Proprietor: A. MENARINI INDUSTRIE FARMACEUTICHE RIUNITE S.R.L., I-50131 Firenze (IT)
(72) Inventor: Giolitti, Alessandro, Firenze (IT); Guidi, Antonio, Firenze (IT); Pasqui, Franco, Campi Bisenzio, Firenze (IT); Pestellini, Vittorio, Firenze (IT); Arcamone, Federico, Nerviano, Milano (IT)
(74) Representative: Mannucci, Gianfranco, Dott.-Ing.

(56) References cited:
- GB-A- 2 144 744
- US-A- 4 697 005
- PATENT ABSTRACTS OF JAPAN vol. 12, no. 467 (C-550)(3314) 07 December 1988, & JP-A-63188674
- CHEMICAL ABSTRACTS, vol. 109, no. 1, 04 July 1988 Columbus, Ohio, USA K.D. Ok et al: "Synthesis of antitumour active 7 -O-[2,6-dideoxy-2-fluoro-alpha-L-talopyranosyl] daunomycinone and adriamycinone."
- Heterocycles vol. 13, 1979, pages 281 - 287; S. Penco et al: "Synthesis of 10-methoxydaunorubicins and of 10[R]-methoxydoxorubicin via opening of an oxirane intermediate."

## Description

The invention refers to new 7-substituted 2β-acetyl-2α,4α,5,12-tetrahydroxy-1,2,3,4-tetrahydro-6,11-naphthacenediones which have a fluorine atom at position 1 or position 3, having the general formula I: 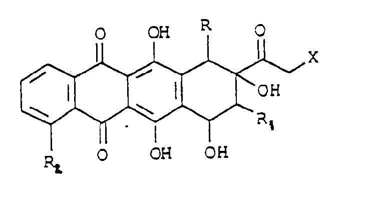
where X is a hydrogen atom or a hydroxyl group; R and R₁, which cannot be equal, are a hydrogen atom or a fluorine atom; R₂ can be a hydrogen atom or a hydroxyl group or an alkoxy group.

The introduction of a fluorine atom instead of a hydrogen atom at position 1 or at position 3 of these naphthacenediones can modify the reactivity of the hydroxyl at 2α and this is of advantage in those cases in which a different reactivity of this hydroxyl can be useful, in particular when the 7-substituted 2β-acetyl-2α,4α,5,12-tetrahydroxy-1,2,3,4-tetrahydro-6,11-naphthacenediones are used as aglycones of anthracyclines having the general formula II: 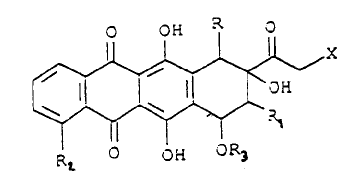
where X, R, R₁ and R₂ are as defined herein above and R₃ is a glycosidic residue. These anthracyclines of general formula II are useful as chemotherapeutic compounds with particular reference to antitumoral and antiviral activity. The invention, therefore, refers also to the use of said compounds for the production of pharmaceutical compositions having antitumoral and antiviral activity.

Further particular compounds of the present invention are set out herein below and in the annexed claims, preferred compounds are listed in claim 7.

The invention also refers to a method of synthesis of use in obtaining the formula: 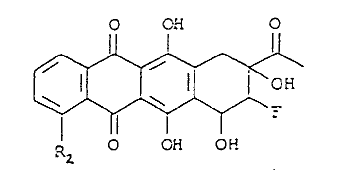
where R₂ is as specified above.

The synthesis used to obtain the substituted naphthacenedione is demonstrated by the following diagram:

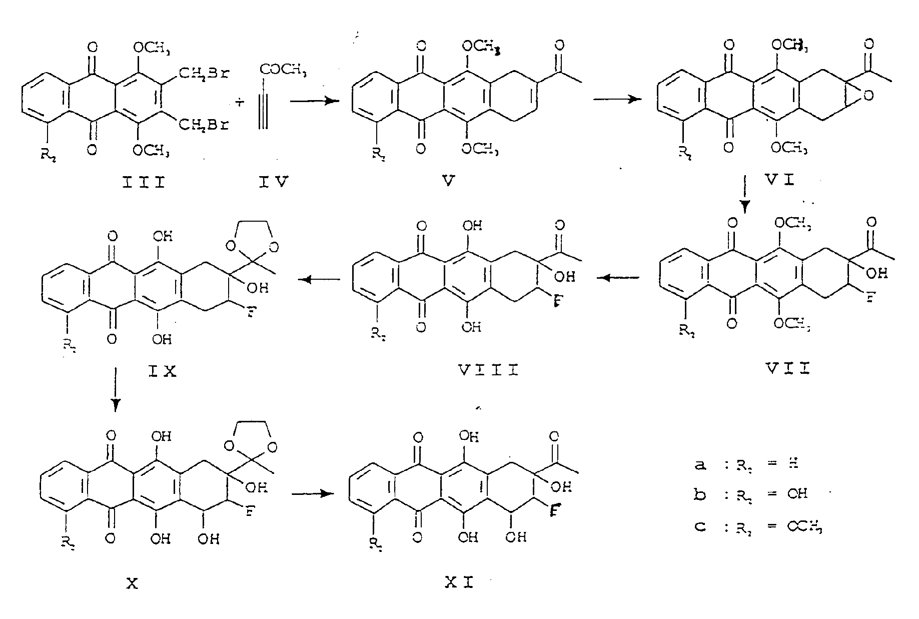

The invention also concerns the abovementioned intermediate products V to XI.

Compound III is obtained as described by F.A.J. Kerdesky, R.D. Ardecky, M.V. Lakshmikantham and M.P. Cava in J. AM. CHEM. SOC. 1981, 103, 1992-1996; or by R.J. Ardecky, D. Dominguer and M.P. Cava in J. ORG. CHEM. 1982, 47, 409-412.

To obtain intermediate compound V a variant from Kerdesky et al. (F.A.J. Kerdesky, R.D. Ardecky, M.V. Lakshmikantham and M.P. Cava, J. AM. CHEM. SOC. 1981, 103, 1992-1996) is used: the cyclizing is carried out by reaction of 3-butin-2-one with 1,4-dimethoxy-2,3-bis(bromomethyl)-anthraquinone, which in the particular reaction conditions acts like a diene. The naphthacenedione is then oxidized on the 2,3 double bond (intermediate compound VI) with a peroxy acid in an appropriate solvent.

To obtain intermediate compound VII the oxiran ring is opened with a nucleophilic fluorine, as for example Olah's reagent.

Demethylation of the phenolic hydroxyls for the purpose of obtaining intermediate compound VIII is carried out, with almost quantitative yield, by using an appropriate Lewis acid, such for example as boron trichloride.

Before brominating position 4 it is preferable to protect the acetyl chain, for example with ethylene glycol (intermediate compound IX). The subsequent bromination at position 4 is performed with an appropriate brominating agent, for example bromine or N-bromo-succinimide or 1,3-dibromo-5,5-dimethyldantoin, or poli(4-vinylpyridin bromohydrate perbromide) carried by a polimer, in a suitable solvent, with or without the presence of radicalic initiators such as light or azo-bis-isobutyronitrile. The bromine derivative obtained can easily undergo substitution by a hydroxyl in order to give intermediate X. This is deprotected in acid medium, thus obtaining 7-substituted 2β-acetyl-2α,4α, 5,12-tetrahydroxy-3β-fluoro-1,2,3,4-tetrahydro-6,11-naphthacenedione(intermediate XI).

The compounds of general formula I where R =F and R₁ =H can be obtained starting from an anthracycline having the general formula: 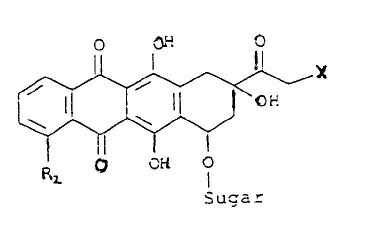
which is oxidated to give compound XII, for example as described in Heterocycles, 13, 281-288 (1979), to continue according to the following diagram:

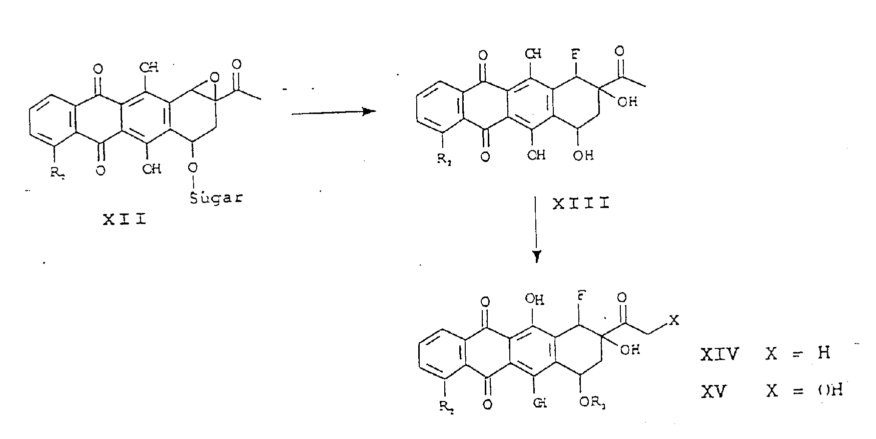

To obtain the naphthacenedione XIII the oxirane ring of intermediate XII, obtained for example as in S. Penco, F. Gozzi, A. Vigevani, M. Ballabio, F. Arcamone, HETEROCYCLES, 13; 1979, 281-288, is opened with a nucleophilic fluorine. The reaction simultaneously causes removal of the glycoside residue, leaving the hydroxyl of the naphthacenedione available for condensation with suitable carbohydrates.

Glycosidation of compound XI or XII to give compounds belonging to the general formula II is performed according to methods described in literature by reaction of said intermediates with a suitably protected carbohydrate derivative presenting a chemical function at position 1 capable, in suitable conditions, of originating a sufficiently stabilized carbocation able to give rise to condensation with the benzyl hydroxyl of the naphthacenedione and formation of a glucoside bond. Typical examples of carbohydrate reagents are 3-N-trifluoroacetyl-1,4-bis(O-p-n-nitrobenzoyl)-daunosamine, 3-N-[(allyloxy)carbonyl]amino-1-(O-p-nitrobenzoyl)-4-O-[(allyloxy)carbonyl]-daunosamine, 3-N-trifluoroacetyl-1,4-bis(O-p-nitrobenzoyl)-acosamine, 3-N-[(allyloxy)carbonyl]amino-1-(O-p-nitrobenzoyl)-4-O-[(allyloxy)carbonyl]-acosamine, 3-N-trifluoroacetyl-1-O-p-nitrobenzoyl-4-deoxy-daunosamine, 3-N-[(allyloxy)carbonyl]amino-1-(O-p-nitrobenzoyl)-4-deoxy-daunosamine, or 3,4-di-trifluoroacetyl-1-chlorodaunosamine, 3,4-di-trifluoroacetyl-1-chloroacosamine, 3-N-[(allyloxy)carbonyl]-4-O-[(allyloxy)carbonyl]-1-chlorodaunosamine, 3-N-[(allyloxy)carbonyl]-4-O-[(allyloxy)carbonyl]-1-chloroacosamine or the corresponding 4-deoxy derivative.

As sugar alcoholic function protective group the trimethylsilyl derivative may also be used, while its position 1 can also be activated as 1-bromo-, 1-fluoro-, 1-trimethylsilyloxy, 1-acetoxy, or 1,2-glycal.

Condensing agents in the reaction between carbohydrat reagent and intermediates XI or XIII may be salts such as silver triflate or silver perchlorate, mercuric oxide and bromide mixtures, trimethylsilyl triflate, or acids such as p-toluenesulfonic acid, or Lewis acids such as a boron trihalogenide, tin tetrachloride or chemical compounds with equivalent properties. The solvents used are generally chlorinated solvents such as methylene chloride, hydrocarbons such as benzene or toluene, ethers such as tetrahydrofuran, or mixtures or these with one another and/or with other solvents such as dimethylformamide, with or without the presence of organic bases such as pyridine, collidine or equivalents.

The protective groups on the carbohydrate residue are removed by suitable basic reactants by procedures known from the literature. In case of allyloxycarbonyl derivatives, this group is removed by the procedure indicated in P. D. Jeffrey and S. W. McCombie, J Org. CHEM., 47 (3), 587-590, (1982, or as described in J Org. CHEM., 38, 3233, (1973), or Tetrahedron Lett., 30, 3773 (1989).

Compounds with X = OH are obtained by starting from hydrochlorides or other appropriate salts of the corresponding compounds with X = H by treatment with bromine in a mixture of solvents, preferably methyl alcohol and dioxane, even though the nature of the solvents, provided that they are compatible with the reaction conditions, cannot limit the significance of the present invention. The bromoacetyl derivative obtained is then replaced by the hydroxyl through a reaction with aqueous solutions of an inorganic base such as an alkali metal hydroxide, or an organic base such as an amine, or a salt such as an alkali metal salt of a weak inorganic acid or an organic acid, for example sodium bicarbonate or sodium formate.

The following examples describe the invention without limiting, however, its scope.

### Example 1:

### Va (2-acetyl-5,12-dimethoxy-1,4-dihydro-6,11-naphthacenedione)

29 g (193 mmoles) of NaI are dissolved under nitrogen in a mixture of 50 ml of dimethylacetamide and 10 ml of dioxane. Dissolution is exothermic: when the temperature has dropped to 40°C, 6.6 g (97 mmoles) of butinone IV are added in a single portion. The temperature of the mixture is brought to 65°C and, over a period of one hour, a solution consisting of 5 g (11 mmoles) of IIIa and 100 ml of dimethylacetamide is added dropwise, keeping the temperature close to the starting value. At the end of the addition stirring is continued at 65-70°C for another hour, and the mixture is then cooled and poured into 600 ml of water. The solid obtained is filtered off, washed well with water and dried under vacuum over P₂O₅ to give 3.85 g of Va. M.P. 209-213°C. ¹H-NMR, δ: 2.40 (s, 3H); 3.60-3.80 (m, 4H); 3.91 (s, 3H); 3.92 (s, 3H) ; 7.00-7.10 (m, 1H); 7.64-7.76 (m, 2H); 8.10-8.22 (m, 2H). Ms, m/z (%): 362 (M⁺, 100); 360 (91); 347 (27); 344 (14); 331 (49); 320 (30); 319 (60).
TLC: n-hexane/THF 2:1 R_{f} = 0.33.

### Example 2:

### Vc (2-acetyl-5,7,12-trimethoxy-1,4-dihydro-6,11-naphthacenedione)

29 g (193 mmoles) of NaI are dissolved under nitrogen in a mixture of 50 ml of dimethylacetamide and 10 ml of dioxane. Dissolution is exothermic: when the temperature has dropped to 40°C, 6.6 g (97 mmoles) of butinone IV are added in a single portion. The temperature of the mixture is brought to 65°C and, over a period of one hour, a solution consisting of 5.3 g (11 mmoles) of IIIc and 100 ml of dimethylacetamide is added dropwise, keeping the temperature close to the starting value. At the end of the addition stirring is continued at 65-70°C for another hour, and the mixture is then cooled and poured into 600 ml of water. The solid obtained is filtered off, washed well with water and dried under vacuum over P₂O₅ to give 3.9 g of crude material. The product Vc is separated by chromatography on a silica column eluting with n-hexane/THF 2:1. 1.9 g of pure product are obtained.
Ms, m/z (%) 392 (M⁺, 100).
TLC: n-hexane/THF 2:1 R_{f} = 0.3.

### Example 3:

### VIa (2-acetyl-2,3-epoxy-5,12-dimethoxy-1,2,3,4-tetrahydro-6,11-naphthacenedione)

3.0 g (8.3 mmoles) of Va and 18 g of 55% mCPBA (3-Chloro-peroxybenzoic acid) (57 mmoles) are refluxed under nitrogen in 400 ml of chloroform for 7 hours. The reaction mixture, cooled to room temperature, is treated with 10% sodium bisulfite solution (2 x 100 ml), then with 5% sodium bicarbonate (3 x 100 ml), and lastly with water. The organic phase is dried over sodium sulfate and the solvent evaporated. The crude reaction material is purified by suspending it in boiling carbon tetrachloride, cooled and filtered off and the solid is washed well with the same solvent to give 850 mg of VIa at 90% purity (yield 30%). M.P. 241-245°C (dec.). ¹H-NMR, δ: 2.16 (s, 3H); 3.00-3.09, 3.55-4.10 (m, 5H); 3.88 (s, 3H); 3.89 (s, 3H); 7.65-7.80 (m, 2H); 8.10-8.20 (m, 2H). MS, m/z (%): 378 (M⁺, 70); 347 (26); 346 (41); 335 (61); 291 (50); 189 (59); 176 (68); 165 (100).

### Example 4:

### VIc (2-acetyl-2,3-epoxy-5,7,12-trimethoxy-1,2,3,4-tetrahydro-6,11-naphthacenedione)

1.9 g (4 mmoles) of Vc and 8.7 g of 55% mCPBA (3-Chloro-peroxybenzoic acid) (27.5 mmoles) are refluxed under nitrogen in 250ml of chloroform for 7 hours. The reaction mixture, cooled to room temperature, is treated with 10% sodium bisulfite solution (2 x 100 ml), then with 5% sodium bicarbonate (3 x 100 ml), and lastly with water. The organic phase is dried over sodium sulfate and the solvent evaporated. The crude reaction material is purified by suspending it in boiling carbon tetrachloride, cooled and filtered off and the solid is washed well with the same solvent to give 600 mg of VIc at 90% purity.
MS, m/z (%): 408 (M⁺, 80).

### Example 5:

### VIIa(2β-acetyl-2α-hydroxy-3β-fluoro-5,12-dimethoxy-1,2,3,4-tetrahydro-6,11-naphthacenedione)

1.4 g (3.7 mmoles) of VIa and 50 ml of 70% HF/pyridine are introduced into a polyethylene flask and reacted for 24 hours at room temperature, in a nitrogenous atmosphere. The reaction mixture is poured onto 600 g of ice, with vigorous stirring, then the solid obtained is filtered off and washed repeatedly with water. This crude material is dried under vacuum over P₂O₅ and suspended in 100 ml of boiling acetone; the suspension is left to cool and then filtered, the solid being washed with a little acetone in order to give 880 mg of VIIa. M.P. 241-245°C dec. (yield: 60%).
¹H-NMR, δ: 2.49 (d, ⁵J_{HF} = 2.6 Hz, 3H); 2.90-3.45 (m, 4H); 3.90 (s, 3H); 3.92 (s, 3H); 4.25 (s, 1H, OH); 4.76 (dt, ²J_{HF} = 49.6 Hz, ³J_{HH app.} = 2.6 Hz); 7.65-7.80 (m, 2H); 8.10-8.25 (m, 2H); MS, m/z (%): 398 (M⁺, 100); 380 (12); 355 (36); 335 (30); 291 (47).

### Example 6:

### VIIc(2β-acetyl-2α-hydroxy-3β-fluoro-5,7,12-trimethoxy-1,2,3,4-tetrahydro-6,11-naphthacenedione)

1 g (2.5 mmoles) of VIc and 30 ml of 70% HF/pyridine are introduced into a polyethylene flask and reacted for 24 hours at room temperature, in a nitrogenous atmosphere. The reaction mixture is poured onto 300 g of ice, with vigorous stirring, then the solid obtained is filtered off and washed repeatedly with water. This crude material is dried under vacuum over P₂O₅ and suspended in 50 ml of boiling acetone; the suspension is left to cool and then filtered, the solid being washed with a little acetone in order to give 600 mg of VIIc.
MS, m/z (%): 428 (M⁺, 100).

### Example 7:

### VIIIa(2β-acetyl-2α,5,12-trihydroxy-3β-fluoro-1,2,3,4-tetrahydro-6,11-naphthacenedione)

82 mg (0.21 mmoles) of VIIa are suspended, under nitrogen, in 15 ml of anhydrous methylene chloride and, after bringing the temperature to -65°C with an acetone and dry ice bath, 3 ml of a 1M solution of boron trichloride (3 mmoles) in methylene chloride are added over a period of 5′. The mixture is stirred for a further 30′ at -65°C before slowly adding 4 ml of methanol and allowing the reaction mixture to return to room temperature. By evaporating the solvent, 79 mg (100%) of VIIIa are finally obtained. M.P. 247-253°C.
¹H-NMR, (DMSO) δ: 2.35 (s, 3H); 2.85-3.25 (m, 4H); 5.26 (d, br, ²J_{HF} = 47.6 Hz); 6.34 (s, br, 1H, OH); 7.85-8.00 (m, 2H); 8.15-8.30 (m, 2H); 13.24 (s, 1H); 13.28 (s, 1H). MS, m/z (%); 370 (M⁺, 24); 352 (55); 332 (63); 327 (33); 307 (100); 187 (80).

### Example 8:

### VIIIc(2β-acetyl-2α,5,12-trihydroxy-3β-fluoro-7-methoxy-1,2,3,4-tetrahydro-6,11-naphthacenedione)

90 mg (0.21 mmoles) of VIIc are suspended, under nitrogen, in 15 ml of anhydrous methylene chloride and, after bringing the temperature to -65°C with an acetone and dry ice bath, 3 ml of a 1M solution of boron trichloride (3 mmoles) in methylene chloride are added over a period of 5′. The mixture is stirred for a further 30′ at -65°C before slowly adding 4 ml of methanol and allowing the reaction mixture to return to room temperature. By evaporating the solvent, 84 mg (100%) of VIIIc are finally obtained.
MS, m/z (%): 400 (M⁺, 35).

### Example 9:

### IXa (2β-[2-methyl-1,3-dioxolanyl-2-yl]2α,5,12-trihydroxy-3β-fluoro-1,2,3,4-tetrahydro-6,11-naphthacenedione)

The following are introduced into a 250-ml flask with a Dean Stark equipment for reaction under reflux, in a nitrogen atmosphere: 320 mg (0.86 mmoles) ofVIIIa and 7.77 g (7 ml, 124 mmoles) of ethylene glycol in 100 ml of benzene, in the presence of 70 mg (0.4 mmoles) of p-toluenesulfonic acid, removing the azeotrope from the reaction medium as it is distilled. At the end the solvent is concentrated to a small volume and the solid obtained is filtered off, washing it successively with a little benzene, ethanol, water, 5% sodium bicarbonate water-solution, and again water. Finally it is dried under vacuum at 80°C to give 283 mg (0.68 mmoles) of IXa: M.P. 237-240°C. (Yield 79.1%). M.P. 239-241°C (toluene).
TLC: CCl₄/AcOEt 2:1 R_{f} = 0.54.

### Example 1O:

### IXc (2β-[2-methyl-1,3-dioxolanyl-2-yl]2α,5,12-trihydroxy-3β-fluoro-7-methoxy-1,2,3,4-tetrahydro-6,11-naphthacenedione)

The following are introduced into a 250-ml flask with a Dean Stark equipment for reaction under reflux, in a nitrogen atmosphere: 350 mg (0.88 mmoles) of VIIIc and 7.77 g (7 ml, 124 mmoles) of ethylene glycol in 100 ml of benzene, in the presence of 70 mg (0.4 mmoles) of p-toluenesulfonic acid, removing the azeotrope from the reaction medium as it is distilled. At the end the solvent is concentrated to a small volume and the solid obtained is filtered off, washing it successively with a little benzene, ethanol, water, 5% sodium bicarbonate water-solution, and again water. Finally it is dried under vacuum at 80°C to give 310 mg (0.68 mmoles) of IXc.
TLC: CCl₄/AcOEt 2:1 R_{f} = 0.5

### Example 11:

### Xa (2β-[2-methyl-1,3-dioxolanyl-2-yl]-2α,4α,5,12-tetrahydroxy-3β-fluoro-1,2,3,4-tetrahydro-6,11-naphthacenedione)

880 mg of IXa are suspended in 200 ml of carbon tetrachloride, under nitrogen, together with 880 mg of potassium carbonate. A solution of 500 mg of bromine in 20 ml of carbon tetrachloride is added. By irradiating with a 500 W lamp the mixture is brought to slight reflux. After about 5′, the product dissolves. The reaction is stopped after 1 hour. The mixture is diluted with 200 ml of chloroform and washed first with bicarbonate and then water. It is rendered anhydrous over sodium sulfate and the solvent is evaporated. The residue is chromatographed on silica, eluting first with carbon tetrachloride/ethyl acetate 3:1 to eliminate the residual starting product and the by-products. It is then eluted with ethyl acetate until 300 mg of Xa is obtained.
¹H-NMR, δ: 1.53 (d, ⁵J_{HF} = 2.2 Hz, 3H); 2.95 (d, ⁴J_{HF} = 2.2, 1H, OH₂); 3.02 (H₁ₐₓ, part B of ABMX spectrum, ²J_{HH} = 18.8 Hz, ⁴J_{HF} = 2.6 Hz); 3.26 (H_{1eq}, part A of ABMX spectrum, ²J_{HH} = 18.8 Hz, ⁴J_{HH} = 0.6 Hz, ⁴J_{HF} = 2.7 Hz); 4.02-4.18 (m, 4H); 3.70 (d, ³J_{HH} = 10.3 Hz, 1H, OH₄); 5.17 (ddd, ³J_{HH} = 10.3 Hz, =³J_{HH} = 2.3, ³J_{HF} = 14.1 Hz, 1H); 5.18 (ddd, ²J_{HF} = 45.6 Hz, ³J_{HH} = 2.3 Hz, ⁴J_{HH} = 0.6 Hz, 1H); 7.80-7.90 (m, 2H); 8.30-8.43 (m, 2H); 13.41 (s, 1H); 13.63 (s, 1H).

### Example 12:

### Xc (2β-[2-methyl-1,3-dioxolanyl-2-yl]-2α,4α,5,12-tetrahydroxy-3β-fluoro-7-methoxy-1,2,3,4-tetrahydro- 6,11-naphthacenedione)

900 mg of IXc are suspended in 200 ml of carbon tetrachloride, under nitrogen, together with 880 mg of potassium carbonate. A solution of 500 mg of bromine in 20 ml of carbon tetrachloride is added. By irradiating with a 500 W lamp the mixture is brought to slight reflux. After about 5′, the product dissolves. The reaction is stopped after 1 hour. The mixture is diluted with 200 ml of chloroform and washed first with bicarbonate and then water. It is rendered anhydrous over sodium sulfate and the solvent is evaporated. The residue is chromatographed on silica, eluting first with carbon tetrachloride/ethyl acetate 3:1 to eliminate the residual starting product and the by-products. It is then eluted with ethyl acetate until 330 mg of Xc is obtained.

### Example 13:

### XIa(2β-acetyl-2α,4α,5,12-tetrahydroxy-3β-fluoro-1,2,3,4-tetrahydro-6,11-naphthacenedione)

82 mg (0.19 mmoles) of Xa are dissolved under nitrogen in 5 ml of anhydrous CH₂Cl₂ and the solution obtained is cooled to 0°C. Then 5 ml (5 mmoles) of a 1M solution of BCl₃ in CH₂Cl₂ are added and the ice-bath is removed. The mixture is stirred at room temperature for a further 2 days, then 15 ml of water are added carefully and, after dilution with 20 ml of CH₂Cl₂, the mixture is washed with water (3 x 20 ml). The organic phase is dried over sodium sulfate, the solvent evaporated and the raw material purified by silica column chromatography (eluent: CHCl₃/acetone 4:1). 40 mg of XIa are obtained. (Yield 50%).
¹H-NMR, δ: 2.54 (d, ⁵J_{HF} = 2.2 Hz, 3H); 3.18 (H₁ₐₓ, ²J_{HH} = 18.5 Hz, ⁴J_{HF} = 2.4 Hz); 3.3 (H_{1eq}, ²J_{HH} = 18.5 Hz, ⁴J_{HF} = 3.1 Hz); 3.65 (bd, ⁴J_{HF} = 2.9, 1H, OH₂) ; 4.6 (bs, 1H, OH₄); 4.98 (dd, =²J_{HF} = 46.2 Hz, J_{HHapp} = 2.2 Hz, 1H); 5.18 (bd, ³J_{HF} = 13 Hz, 1H); 7.80-7.90 (m, 2H); 8.20-8.43 (m, 2H); 13.29 (s, 1H); 13.53 (s, 1H).

### Example 14:

### XIc(2β-acetyl-2α,4α,5,12-tetrahydroxy-3β-fluoro-7-methoxy-1,2,3,4-tetrahydro-6,11-naphthacenedione)

88 mg (0.19 mmoles) of Xc are dissolved under nitrogen in 5 ml of anhydrous CH₂Cl₂ and the solution obtained is cooled to 0°C. Then 5 ml (5 mmoles) of a 1M solution of BCl₃ in CH₂Cl₂ are added and the ice-bath is removed. The mixture is stirred at room temperature for a further 2 days, then 15 ml of water are added carefully and, after dilution with 20 ml of CH₂Cl₂, the mixture is washed with water (3 x 20 ml). The organic phase is dried over sodium sulfate, the solvent evaporated and the raw material purified by silica column chromatography (eluent: CHCl₃/acetone 4:1). 50 mg of XIc are obtained.
MS, m/z (%): 462 (M⁺, 100).

### Example 15:

### (4-demethoxy-8-fluoro-daunomycin)

142 mg of (-)-3-N-trifluoroacetyl-1,4-bis(O-p-nitrobenzoyl)-1-daunosamine are suspended in a solution of 45 mg of XIa in 8.3 ml of anhydrous methylene chloride and 7 ml of anhydrous ether, under nitrogen, in the presence of 600 mg of granular 4 Å molecular sieves. The mixture is cooled to 0°C and 0.08 ml of trimethylsilyl triflate added. After three hours the reaction mixture is treated with 50 ml of ethyl acetate and 100 ml of saturated sodium bicarbonate solution, and the organic phase is then washed with sodium chloride solution. After rendering anhydrous, the solvent is evaporated and the residue purified by silica chromatography, eluting with chloroform/acetone 4:1. 80 mg of product are obtained, which are dissolved in 0.6 ml of methylene chloride and 37 ml of methanol. The solution is cooled to 0°C and, in a nitrogen atmosphere, 1.2 ml of 0.1M sodium hydroxide are added. The solution is stirred for 30 minutes, and the glacial acetic acid is added until the color of the solution becomes pale orange. It is treated with 60 ml of ethyl acetate and 60 ml of sodium chloride solution. The organic phase is washed twice more with sodium chloride solution, then rendered anhydrous and evaporated. The residue is dissolved in 15 ml of 0.1M sodium hydroxide at 0°C and stirred for 20 minutes in a nitrogen atmosphere. The pH of the solution is brought to 8 with 5M HCl, and the solution is then extracted repeatedly with chloroform. The organic phase is washed with water, rendered anhydrous and concentrated under vacuum. The residue is dissolved in a little chloroform and methanol (9:1), HCl in 0.25M methanol added until the pH is 3.5, and then either is added until the hydrochloride of the requested product precipitates.
TLC: (free base) R_{f} = 0.52 (CHCl₃/MeOH/H₂O 13:6:1).

### Example 16:

### 8-fluoro-daunomycin

142 mg of (-)-3-N-trifluoroacetyl-1,4-bis(O-p-nitrobenzoyl)-1-daunosamine are suspended in a solution of 48 mg of XIc in 8.3 ml of anhydrous methylene chloride and 7 ml of anhydrous ether, under nitrogen, in the presence of 600 mg of granular 4 Å molecular sieves. The mixture is cooled to 0°C and 0.08 ml of trimethylsilyl triflate added. After three hours the reaction mixture is treated with 50 ml of ethyl acetate and 100 ml of saturated sodium bicarbonate solution, and the organic phase is then washed with sodium chloride solution. After rendering anhydrous, the solvent is evaporated and the residue purified by silica chromatography, eluting with chloroform/acetone 4:1. 85 mg of product are obtained, which are dissolved in 0.6 ml of methylene chloride and 37 ml of methanol. The solution is cooled to 0°C and, in a nitrogen atmosphere, 1.2 ml of 0.1M sodium hydroxide are added. The solution is stirred for 30 minutes, and then glacial acetic acid is added until the color of the solution becomes pale orange. It is treated with 60 ml of ethyl acetate and 60 ml of sodium chloride solution. The organic phase is washed twice more with sodium chloride solution, then rendered anhydrous and evaporated. The residue is dissolved in 15 ml of 0.1M sodium hydroxide at 0°C and stirred for 20 minutes in a nitrogen atmosphere. The pH of the solution is brought to 8 with 5M HCl, and the solution is then extracted repeatedly with chloroform. The organic phase is washed with water, rendered anhydrous and concentrated under vacuum. The residue is dissolved in a little chloroform and methanol (9:1), HCl in 0.25M methanol added until the pH is 3.5, and then ether is added until the hydrochloride of the desired product precipitates.
TLC: (free base) R_{f} = 0.49 (CHCl₃/MeOH/H₂O 13:6:1).

### Example 17:

### XIIIc (1 -Fluoro-2β-acetyl-2α,4α,5,12-tetrahydroxy-1,2,3,4-tetrahydro-6,11-naphthacenedione)

1 g of XIIc are treated at room temperature with 15 ml of 70% strength HF/pyridine (Olah's reagent) with stirring. After 1 hour the mixture is poured onto ice, the product is extracted with methylene chloride and the organic phase washed well with water. It is rendered anhydrous, the solvent is evaporated off, and the product is purified by chromatography on silica, eluting with chloroform/acetone 4:1.

TLC (chloroform/acetone 4:1): R_{f} = 0.47.
¹H-NMR, δ: 1.53 (d, ⁵J_{HF} = 2.2 Hz, 3h); 2,95 (d, ⁴J_{HF} = 2.2, 1H, OH₂); 3.02 (H₁ₐₓ, part B of ABMX spectrum, ²J_{HH} = 18.8 Hz, ⁴J_{HF} = 2.6 Hz); 3.26 (H_{1eq}, part A of ABMX spectrum, ²J_{HH} = 18.8 Hz, ⁴J_{HH} = 0.6 Hz, ⁴J_{HF} = 2.7 Hz); 4.02-4.18 (m, 4H); 3.70 (d, ³J_{HH} = 10.3 Hz, 1H, OH₄); 5.17 (ddd, ³J_{HH} = 10.3 Hz, ³J_{HH} = 2.3, ³J_{HF} = 14.1 Hz, 1H); 5.18 (ddd, ²J_{HF} = 45.6 Hz, ³J_{HH} = 2.3 Hz, ⁴J_{HH} = 0.6 Hz, 1H); 7.80-7.90 (m, 2H); 8.30-8.43 (m, 2H); 13.41 (s, 1H) 13.63 (s, 1H).

### Example 18:

### (10 -Fluoro-4′-epi-daunomycin)

280 mg (0.60 mol) of (-)-3-n-[(allyloxy)carbonyl]amino-1-(O- p-nitrobenzoyl)-4-O-[(allyloxy)carbonyl]-1-acosamine and 110 mg of XIIIc are dissolved in 24 ml of anhydrous methylene chloride and 20 ml of anhydrous ether, under nitrogen, in the presence of granular 4Å molecular sieves. The mixture is cooled to 0°C and 0.22 ml of O-trimethylsilsyl trifluoromethanesulfonate are added with stirring. After two hours the reaction mixture is diluted with methylene chloride and the organic phase is washed with saturated sodium bicarbonate solution then with water. It is rendered anhydrous and the solvent evaporated off.

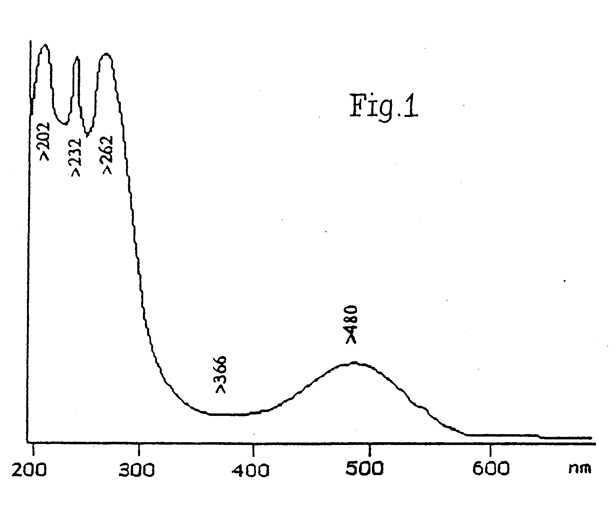

The residue is dissolved in 30 ml of ethyl acetate and 40 mg of palladium tetrakis-(triphenylphosphine), 40 mg of triphenylphosphine and 350 mg of 2-ethylhexanoic acid are then added in a nitrogen atmosphere and at room temperature. The solution is stirred for 24 hours, then diluted with methylene chloride, and next washed with saturated sodium bicarbonate solution and then water. It is rendered anhydrous and the solvent is evaporated off. The residue is dissolved in a little chloroform and methanol (9:1), 0.25 M HCl in methanol is added until the pH is 3.5, and then ether is added until the hydrochloride of the desired product precipitates.
TCL (chloroform/acetone 6:1): R_{f} = 0.2
The UV spectrum is shown in Fig. 1.

### Example 19:

### (10 -Fluoro-4′-epi-doxorubicin)

The compound obtained as in the example 18 is converted by the procedure indicated in the reference cited (Arcamone, 1981, p 22), obtaining, in a yield of approx. 60%, 10 - fluoro-4′-epi-doxorubicin, isolated in hydrochloride form.

The compounds which form the subject of this invention were evaluated "in vitro" in human tumor cell cultures and "in vivo" against transplantable tumors of the mouse according to the methods described in CANCER CHEMIOTHERAPY REPORTS, Part 3, Vol. 3, pp. 9 (1972).

The compounds exhibited greater activity than that of daunorubicin and doxorubicin used as reference products.

## Claims

1. New 7-substituted fluoro-2β-acetyl-2α,4α,5,12-tetrahydroxy-1,2,3,4-tetrahydro-6,11-naphthacenediones having the general formula I: 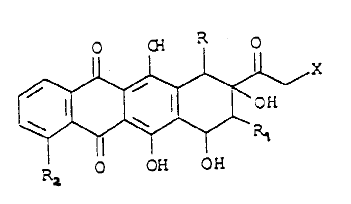 where X is a hydrogen atom or a hydroxyl group; R and R₁, which cannot be equal, are a hydrogen atom or a fluorine atom; R₂ can be a hydrogen atom or a hydroxyl group or an alkoxy group.

2. New 7-substituted fluoro-2β-acetyl-2α,4α,5,12-tetrahydroxy-1,2,3,4-tetrahydro-6,11-naphthacenediones as claimed in claim 1, useful for obtaining new anthracyclinic derivatives for use as chemotherapeutic compounds with particular reference to the antitumoral and antiviral activity.

3. Derivatives obtained from 7-substituted fluoro-2β-acetyl-2α,4α,5,12-tetrahydroxyl-1,2,3,4-tetrahydro-6,11-naphthacenediones as claimed in claim 1 or 2, having the general formula: 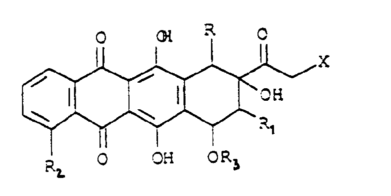 where X, R, R₁, R₂ are as defined in claim 1, and R₃ is a glycosidic residue.

4. Derivatives as claimed in claim 3, as new chemotherapeutic compound with particular reference to antitumoral and antiviral activity.

5. Derivatives as claimed in claim 3, having the general formula: 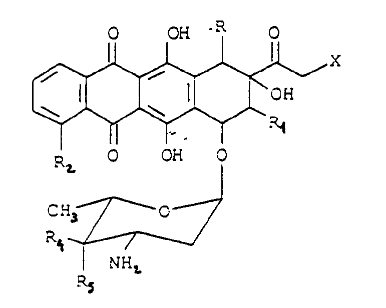 where X is a hydrogen atom or a hydroxyl group; R and R₁, which cannot be equal, are a hydrogen atom or a fluorine atom; R₂ can be a hydrogen atom or a hydroxyl group or an alkoxy group; R₄ and R₅ can be a hydrogen atom or a hydroxyl group, but cannot be simultaneously a hydroxyl group.

6. Derivatives as claimed in claim 5, as new chemotherapeutic compounds with particular reference to antitumoral and antiviral activity.

7. A derivative as claimed in claim 5, chosen from the following:
8-fluoro-4-demethoxydaunorubicin or a pharmaceutically acceptable salt thereof;
8-fluoro-4-demethoxydoxorubicin or a pharmaceutically acceptable salt thereof;
8-fluoro-4-demethoxy-4′-epi-daunorubicin or a pharmaceutically acceptable salt thereof;
8-fluoro-4-demethoxy-4′-epi-doxorubicin or a pharmaceutically acceptable salt thereof;
8-fluoro-4-demethoxy-4′-deoxy-daunorubicin or a pharmaceutically acceptable salt thereof;
8-fluoro-4-demethoxy-4′-deoxy-doxorubicin or a pharmaceutically acceptable salt thereof;
8-fluoro-carminomycin or a pharmaceutically acceptable salt thereof;
8-fluoro-14-hydroxy-carminomycin or a pharmaceutically acceptable salt thereof;
8-fluoro-4′-epi-carminomycin or a pharmaceutically acceptable salt thereof;
8-fluoro-14-hydroxy-4′-epi-carminomycin or a pharmaceutically acceptable salt thereof;
8-fluoro-4′-deoxy-carminomycin or a pharmaceutically acceptable salt thereof;
8-fluoro-14-hydroxy-4′-deoxy-carminomycin or a pharmaceutically acceptable salt thereof;
8-fluoro-daunorubicin or a pharmaceutically acceptable salt thereof;
8-fluoro-doxorubicin or a pharmaceutically acceptable salt thereof;
8-fluoro-4′-epi-daunorubicin or a pharmaceutically acceptable salt thereof;
8-fluoro-4′-epi-doxorubicin or a pharmaceutically acceptable salt thereof;
8-fluoro-4′-deoxy-daunorubicin or a pharmaceutically acceptable salt thereof;
8-fluoro-4′-deoxy-doxorubicin or a pharmaceutically acceptable salt thereof;
10-fluoro-4-demethoxydaunorubicin or a pharmaceutically acceptable salt thereof;
10-fluoro-4-demethoxydoxorubicin or a pharmaceutically acceptable salt thereof;
10-fluoro-4-demethoxy-4′-epi-daunorubicin or a pharmaceutically acceptable salt thereof;
10-fluoro-4-demethoxy-4′-epi-doxorubicin or a pharmaceutically acceptable salt thereof;
10-fluoro-4-demethoxy-4′-deoxy-daunorubicin or a pharmaceutically acceptable salt thereof;
10-fluoro-4-demethoxy-4′-deoxy-doxorubicin or a pharmaceutically acceptable salt thereof;
10-fluoro-carminomycin or a pharmaceutically acceptable salt thereof;
10-fluoro-14-hydroxy-carminomycin or a pharmaceutically acceptable salt thereof;
10-fluoro-4′-epi-carminomycin or a pharmaceutically acceptable salt thereof;
10-fluoro-14-hydroxy-4′-epi-carminomycin or a pharmaceutically acceptable salt thereof;
10-fluoro-4′-deoxy-carminomycin or a pharmaceutically acceptable salt thereof;
10-fluoro-14-hydroxy-4′-deoxy-carminomycin or a pharmaceutically acceptable salt thereof;
10-fluoro-daunorubicin or a pharmaceutically acceptable salt thereof;
10-fluoro-doxorubicin or a pharmaceutically acceptable salt thereof;
10-fluoro-4′-epi-daunorubicin or a pharmaceutically acceptable salt thereof;
10-fluoro-4′-epi-doxorubicin or a pharmaceutically acceptable salt thereof;
10-fluoro-4′-deoxy-daunorubicin or a pharmaceutically acceptable salt thereof;
10-fluoro-4′-deoxy-doxorubicin or a pharmaceutically acceptable salt thereof.

8. A naphthacenedione having the formula: 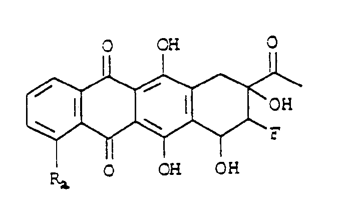 where R₂ is a hydrogen atom or a hydroxyl group or an alkoxy group.

9. A naphthacenedione having the formula: 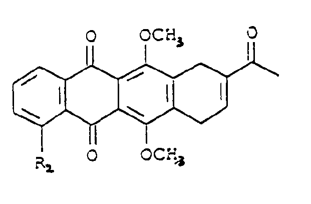 where R₂ is a hydrogen atom or a hydroxyl group or an alkoxy group.

10. A naphthacenedione having the formula: 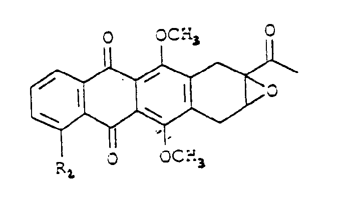 where R₂ is a hydrogen atom or a hydroxyl group or an alkoxy group.

11. A naphthacenedione having the formula: 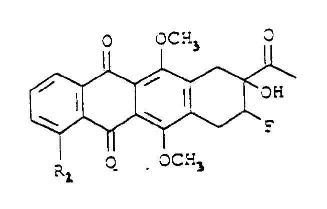 where R₂ is a hydrogen atom or a hydroxyl group or an alkoxy group.

12. A naphthacenedione having the formula: 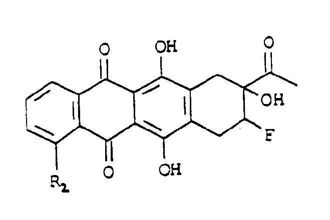 where R₂ is a hydrogen atom or a hydroxyl group or an alkoxy group.

13. A naphthacenedione having the formula: 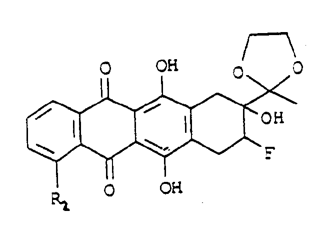 where R₂ is a hydrogen atom or a hydroxyl group or an alkoxy group.

14. A naphthacenedione having the formula: 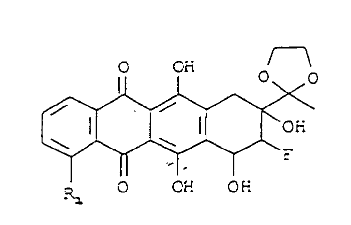 where R₂ is a hydrogen atom or a hydroxyl group or an alkoxy group.

15. Use of one or more of the compounds as claimed in claim 3 or 5 or pharmaceutically acceptable salts thereof, in combination with a pharmaceutically acceptable diluent or vector, for the production of a pharmaceutical composition useful as a chemotherapeutic agent with particular reference to antitumoral and antiviral activity.

16. A process for preparing the compounds as claimed in claim 1, when R=H and R₁=F, such process providing for:
i) cyclization of 1,4-dimethoxy-2,3-bis(bromomethyl)-anthraquinone suitably substituted with 3-butin-2-one with or without the presence of a suitable catalyst;
ii) epoxidation with a peroxy compound;
iii) opening of the oxiran ring with a nucleophilic fluorine;
iv) demethylation of the phenolic hydroxyls and protection of the acetyl chain;
v) brominating of the naphthacenedione at position 4 with bromine or other suitable brominating agent, such as N-bromo succinimide or 1,3-dibromo-5,5-dimethyldantoin, or poli(4-vinylpyridin bromohydrate perbromide) carried by a polymer; subsequent substitution with a hydroxyl; and finally deprotection of the acetyl group.

17. A process as claimed in claim 16, for obtaining the compounds as claimed in claim 1, with X=OH, wherein a bromine atom is introduced on the acetyl group of the 7-substituted fluoro-2β-acetyl-1,2-epoxy-4α,5,12-trihydroxy-1,2,3,4-tetrahydro-6,11-naphthacenedione, and subsequently substituted with a hydroxyl either by means of direct hydrolysis of the bromine derivative or by hydrolysis of a suitable ester, such as formate obtained from the same through exchange reaction; or the hydroxyl is maintained in a suitably protected form.

18. Process for preparing the compounds as claimed in claim 1, when R=F and R₁=H, such process providing for opening of the oxiran ring of the corresponding 7-substituted 2β-acetyl-1,2-epoxy-4α,5,12-trihydroxy-1,2,3,4-tetrahydro-6,11-naphtha-cenedione with a nucleophilic fluorine.

19. Process for preparing the compounds as claimed in claim 3 or 5, such process providing for glycosidation of the compounds obtained by the process of claim 16 or 17 or 18 , with a suitably protected glucidic derivative in the presence of condensing agents, such as silver triflate or silver perchlorate, mercuric oxide and bromide mixtures, trimethylsilyltriflate, or acids such as p-toluensulfonic acid or Lewis acids such as a boron trihalogenide, tin tetrachloride or chemical compounds with equivalent properties, with or without the presence of a suitable dehydrating agent.

20. Process as claimed in claim 19, wherein the deprotection of the obtained compound is performed with basic agents if the protective group is trifluoroacetyl, or with nickel or palladium complexes if the protecting group is allyloxycarbonyl.

21. Process for preparing the compounds as claimed in claim 3, starting from 7-substituted 4-glycosilated 2β-acetyl-2α,4α,5,12-tetradydroxy-1,2,3,4-tetrahydro-6,11-naphthacenediones through the introduction of a bromine atom on the acetyl group at position 2 and substitution with a hydroxyl either by means of direct hydrolysis of the bromine derivative or hydrolysis of a suitable ester, such as formate, obtained by the same through an exchange reaction.

## Patentansprüche

1. Neue 7-substituierte Fluor-2β-acetyl-2α-4α,5,12-tetrahydroxy-1,2,3,4-tetrahydro-6,11-naphthacendione der allgemeinen Formel I: 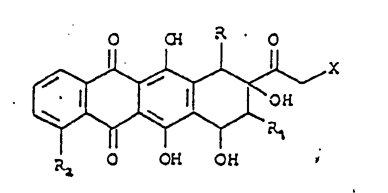 in der X ein Wasserstoffatom oder eine Hydroxylgruppe, R und R₁, die nicht gleich sein können, ein Wasserstoff- oder ein Fluoratom sowie R₂ ein Wasserstoffatom, eine Hydroxylgruppe oder eine Alkoxygruppe bedeuten.

2. Neue 7-substituierte Fluor-2β-acetyl-2α,4α,5,12-tetrahydroxy-1,2,3,4-tetrahydro-6,11-naphthacendione nach Anspruch 1, verwendbar zum Erhalt neuer Anthracyclin-Derivate zur Verwendung als chemotherapeutische Verbindungen, insbesondere im Hinblick auf eine antitumorale und antivirale Aktivität.

3. Derivate, erhalten aus 7-substituierten Fluor-2β-acetyl-2α,4α,5,12-tetrahydroxy-1,2,3,4-tetrahydro-6,11-naphthacendionen gemäß Anspruch 1 oder 2 mit der allgemeinen Formel: 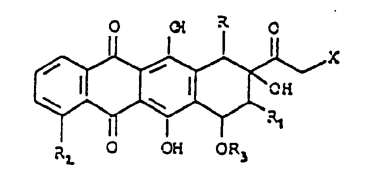 in der X, R, R₁ und R₂ wie in Anspruch 1 definiert sind und R₃ ein glycosidischer Rest ist.

4. Derivate nach Anspruch 3 als neue chemotherapeutische Verbindungen, insbesondere im Hinblick auf eine antitumorale und antivirale Aktivität.

5. Derivate nach Anspruch 3 mit der allgemeinen Formel: 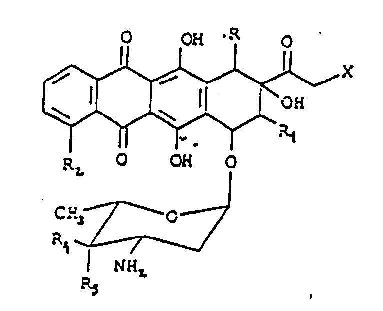 in der X ein Wasserstoffatom oder eine Hydroxylgruppe, R und R₁, die nicht gleich sein können, ein Wasserstoff- oder ein Fluoratom und R₂ ein Wasserstoffatom, eine Hydroxylgruppe oder eine Alkoxygruppe sind sowie R₄ und R₅ ein Wasserstoffatom oder eine Hydroxylgruppe bedeuten, jedoch nicht gleichzeitig eine Hydroxylgruppe sein können.

6. Derivate nach Anspruch 5 als neue chemotherapeutische Verbindungen, insbesondere im Hinblick auf eine antitumorale und antivirale Aktivität.

7. Derivat nach Anspruch 5, ausgewählt aus den folgenden Verbindungen:
8-Fluor-4-demethoxydaunorubicin oder ein pharmazeutisch verträgliches Salz desselben;
8-Fluor-4-demethoxydoxorubicin oder ein pharmazeutisch verträgliches Salz desselben;
8-Fluor-4-demethoxy-4′-epi-daunorubicin oder ein pharmazeutisch verträgliches Salz desselben;
8-Fluor-4-demethoxy-4′-epi-doxorubicin oder ein pharmazeutisch verträgliches Salz desselben;
8-Fluor-4-demethoxy-4′-deoxy-daunorubicin oder ein pharmazeutisch verträgliches Salz desselben;
8-Fluor-4-demethoxy-4′-deoxy-doxorubicin oder ein pharmazeutisch verträgliches Salz desselben;
8-Fluor-carminomycin oder ein pharmazeutisch verträgliches Salz desselben;
8-Fluor-14-hydroxy-carminomycin oder ein pharmazeutisch verträgliches Salz desselben;
8-Fluor-4′-epi-carminomycin oder ein pharmazeutisch verträgliches Salz desselben;
8-Fluor-14-hydroxy-4′-epi-carminomycin oder ein pharmazeutisch verträgliches Salz desselben;
8-Fluor-4′-deoxy-carminomycin oder ein pharmazeutisch verträgliches Salz desselben;
8-Fluor-14-hydroxy-4′-deoxy-carminomycin oder ein pharmazeutisch verträgliches Salz desselben;
8-Fluor-daunorubicin oder ein pharmazeutisch verträgliches Salz desselben;
8-Fluor-doxorubicin oder ein pharmazeutisch verträgliches Salz desselben;
8-Fluor-4′-epi-daunorubicin oder ein pharmazeutisch verträgliches Salz desselben;
8-Fluor-4′-epi-doxorubicin oder ein pharmazeutisch verträgliches Salz desselben;
8-Fluor-4′-deoxy-daunorubicin oder ein pharmazeutisch verträgliches Salz desselben;
8-Fluor-4′-deoxy-doxorubicin oder ein pharmazeutisch verträgliches Salz desselben;
10-Fluor-4-demethoxydaunorubicin oder ein pharmazeutisch verträgliches Salz desselben;
10-Fluor-4-demethoxydoxorubicin oder ein pharmazeutisch verträgliches Salz desselben;
10-Fluor-4-demethoxy-4′-epi-daunorubicin oder ein pharmazeutisch verträgliches Salz desselben;
10-Fluor-4-demethoxy-4′-epi-doxorubicin oder ein pharmazeutisch verträgliches Salz desselben;
10-Fluor-4-demethoxy-4′-deoxy-daunorubicin oder ein pharmazeutisch verträgliches Salz desselben;
10-Fluor-4-demethoxy-4′-deoxy-doxorubicin oder ein pharmazeutisch verträgliches Salz desselben;
10-Fluor-carminomycin oder ein pharmazeutisch verträgliches Salz desselben;
10-Fluor-14-hydroxy-carminomycin oder ein pharmazeutisch verträgliches Salz desselben;
10-Fluor-4′-epi-carminomycin oder ein pharmazeutisch verträgliches Salz desselben;
10-Fluor-14-hydroxy-4′-epi-carminomycin oder ein pharmazeutisch verträgliches Salz desselben;
10-Fluor-4′-deoxy-carminomycin oder ein pharmazeutisch verträgliches Salz desselben;
10-Fluor-14-hydroxy-4′-deoxy-carminomycin oder ein pharmazeutisch verträgliches Salz desselben;
10-Fluor-daunorubicin oder ein pharmazeutisch verträgliches Salz desselben;
10-Fluor-doxorubicin oder ein pharmazeutisch verträgliches Salz desselben;
10-Fluor-4′-epi-daunorubicin oder ein pharmazeutisch verträgliches Salz desselben;
10-Fluor-4′-epi-doxorubicin oder ein pharmazeutisch verträgliches Salz desselben;
10-Fluor-4′-deoxy-daunorubicin oder ein pharmazeutisch verträgliches Salz desselben;
10-Fluor-4′-deoxy-doxorubicin oder ein pharmazeutisch verträgliches Salz desselben.

8. Naphthacendion der Formel: 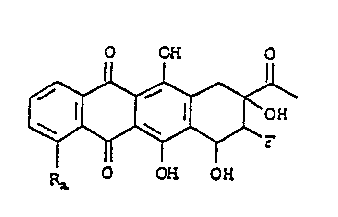 in der R₂ ein Wasserstoffatom, eine Hydroxylgruppe oder eine Alkoxygruppe ist.

9. Naphthacendion der Formel: 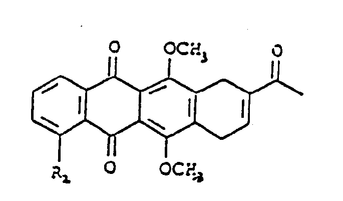 in der R₂ ein Wasserstoffatom, eine Hydroxylgruppe oder eine Alkoxygruppe ist.

10. Naphthacendion der Formel: 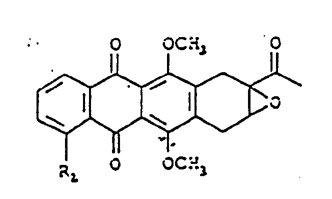 in der R₂ ein Wasserstoffatom, eine Hydroxylgruppe oder eine Alkoxygruppe ist.

11. Naphthacendion der Formel: 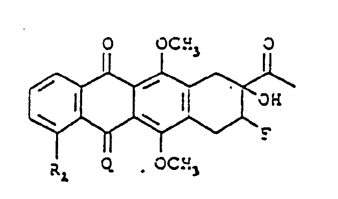 in der R₂ ein Wasserstoffatom, eine Hydroxylgruppe oder eine Alkoxygruppe ist.

12. Naphthacendion der Formel: 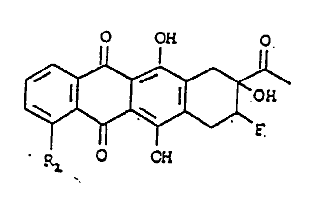 in der R₂ ein Wasserstoffatom, eine Hydroxylgruppe oder eine Alkoxygruppe ist.

13. Naphthacendion der Formel: in der R₂ ein Wasserstoffatom, eine Hydroxylgruppe oder eine Alkoxygruppe ist.

14. Naphthacendion der Formel: 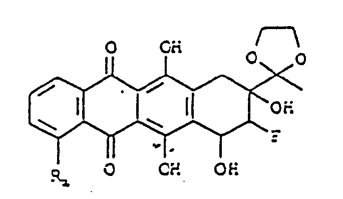 in der R₂ ein Wasserstoffatom, eine Hydroxylgruppe oder eine Alkoxygruppe ist.

15. Verwendung einer Verbindung nach den Ansprüchen 3 oder 5 oder mehrerer oder pharmazeutisch verträglicher Salze derselben in Kombination mit einem pharmazeutisch verträglichen Verdünnungsmittel oder Vektor zur Herstellung einer pharmazeutischen Zusammensetzung für den Einsatz als ein chemotherapeutisches Mittel, insbesondere im Hinblick auf eine antitumorale und antivirale Aktivität.

16. Verfahren zur Herstellung der Verbindungen nach Anspruch 1, wobei R=H und R₁=F ist, mit
i) einer Cyclisierung von geeigenet substituiertem 1,4-Dimethoxy-2,3-bis(brommethyl)-anthrachinon mit 3-Butin-2-on, mit oder ohne Anwesenheit eines geeigneten Katalysators;
ii) einer Epoxidation mit einer Peroxyverbindung;
iii) einer Öffnung des Oxiranringes mit nucleophilem Fluor;
iv) einer Demethylierung der phenolischen Hydroxylgruppen und einem Schutz der Acetylkette;
v) einer Bromierung des Naphthacendions in der Position 4 mit Brom oder einem anderen geeigneten Bromierungsmittel, wie N-Bromsuccinimid, 1,3-Dibrom-5,5-dimethyldantoin oder Poly(4-vinyl-pyridin-bromhydrat-perbromid) auf einem Polymer; einer anschließenden Substitution mit einer Hydroxylgruppe und schließlich einer Entschützung der Acetylgruppe.

17. Verfahren nach Anspruch 16 zum Erhalt der Verbindungen nach Anspruch 1, wobei X=OH ist, wobei ein Bromatom in die Acetylgruppe des 7-substituierten Fluor-2β-acetyl-1,2- epoxy-4α,5,12-trihydroxy-1,2,3,4-tetrahydro-6,11-naphthacendion eingeführt und anschließend mit einer Hydroxylgruppe substituiert wird, und zwar entweder mittels einer direkten Hydrolyse des Bromderivats oder durch Hydrolyse eines geeigneten Esters wie einem Formiat, erhalten aus demselben durch die gleiche Austauschreaktion, oder die Hydroxylgruppe in einer geeignet geschützten Form beibehalten wird.

18. Verfahren zur Herstellung der Verbindungen nach Anspruch 1, wobei R=F und R₁=H ist, wobei der Oxiranring des entsprechenden 7-substituierten 2β-Acetyl-1,2-epoxy-4α,5,12-trihydroxy-1,2,3,4-tetrahydro-6,11-naphthacendions mit nucleophilem Fluor geöffnet wird.

19. Verfahren zur Herstellung der Verbindungen nach Anspruch **3** oder 5, wobei die Verbindungen, erhalten mittels der Verfahren der Ansprüche 16, 17 oder 18, glycosidiert werden, und zwar mit einem geeignet geschützten Glucidderivat in der Gegenwart von Kondensationsmitteln wie Silbertriflat oder Silberperchlorat, Quecksilberoxid und -bromid-Gemischen, Trimethylsilyltriflat oder Säuren wie p-Toluolsulfonsäure oder Lewissäuren wie Bortrihalogeniden, Zinntetrachlorid oder chemischen Verbindungen mit äquivalenten Eigenschaften, mit oder ohne Anwesenheit eines geeigeneten Dehydratisierungsmittels.

20. Verfahren nach Anspruch 19, bei dem die Entschützung der erhaltenen Verbindung mit basischen Mitteln durchgeführt wird, wenn die Schutzgruppe Trifluoracetyl ist, oder mit Nickel- oder Palladiumkomplexen, wenn die Schutzgruppe Allyloxycarbonyl ist.

21. Verfahren zur Herstellung der Verbindungen nach Anspruch 3, wobei man von 7-substituierten 4-glycosierten 2β-Acetyl-2α,4α,5,12-tetrahydroxy-1,2,3,4-tetrahydro-6,11-naphthacendionen unter Einführung eines Bromatoms an der Acetylgruppe in Position 2 ausgeht und dieses mit einer Hydroxylgruppe substituiert, und zwar entweder mittels einer direkten Hydrolyse des Bromderivates oder einer Hydrolyse eines geeigneten Esters, z.B. eines Formiats, erhalten auf gleichem Wege über eine Austauschreaktion.

## Revendications

1. Nouvelles 7-substituées 2β-acéty1-2α,4α,5,12-tétrahydroxy-1,2,3,4-tétrahydro-6,11-naphtacènediones de formule générale I : 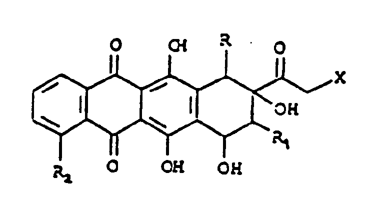 dans laquelle X est un atome hydrogène ou un groupe hydroxyle ; R et R₁ qui ne peuvent être égaux représentent un atome hydrogène ou un atome fluor ; R₂ peut être un atome hydrogène ou un groupe hydroxyle ou un groupe alcoxy.

2. Nouvelles 7-substituées 2β-acétyl-2α,4α,5,12-tétrahydroxy-1,2,3,4-tétrahydro-6,11-naphtacènediones selon la revendication 1, utiles pour l'obtention de nouveaux dérivés anthracycliniques pour l'utilisation comme composés chimiothérapeutiques tout particulièrement vis-à-vis de l'activité antitumorale et antivirale.

3. Dérivés obtenus à partir de 7-substituées 2β-acétyl-2α,4α,5,12-tétrahydroxy-1,2,3,4-tétrahydro-6,11-naphtacènediones selon la revendication 1 ou 2, de formule générale : 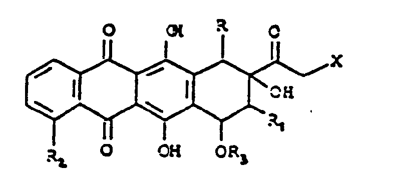 dans laquelle X, R, R₁ et R₂ sont tels que définis dans la revendication 1 et R₃ est un résidu glycosidique.

4. Dérivés selon la revendication 3, en tant que nouveaux composés chimiothérapeutiques tout particulièrement à l'égard de l'activité antitumorale et antivirale.

5. Dérivés selon la revendication 3, de formule générale 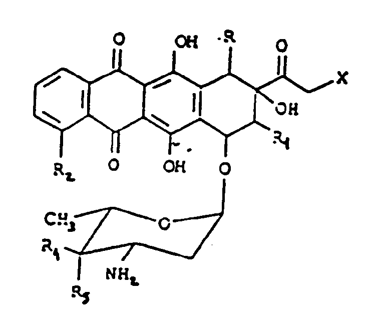 dans laquelle X est un atome hydrogène ou un groupe hydroxyle ; R et R₁ qui ne peuvent être égaux sont un atome hydrogène ou un atome fluor ; R₂ peut être un atome hydrogène ou un groupe hydroxyle ou un groupe alcoxy ; R₄ et R₅ peuvent être un atome hydrogène ou un groupe hydroxyle mais ne peuvent être simultanément un groupe hydroxyle.

6. Dérivés selon la revendication 5, en tant que nouveaux composés chimiothérapeutiques tout particulièrement à l'égard de l'activité antitumorale et antivirale.

7. Dérivé selon la revendication 5, choisi parmi ce qui suit :
8-fluoro-4-déméthoxydaunorubicine ou son sel pharmaceutiquement acceptable ;
8-fluoro-4-déméthoxydoxorubicine ou son sel pharmaceutiquement acceptable ;
8-fluoro-4-déméthoxy-4′-épi-daunorubicine ou son sel pharmaceutiquement acceptable ;
8-fluoro-4-déméthoxy-4′-épi-doxorubicine ou son sel pharmaceutiquement acceptable ;
8-fluoro-4-déméthoxy-4′-déoxy-daunorubicine ou son sel pharmaceutiquement acceptable ;
8-fluoro-4-déméthoxy-4′-déoxy-doxorubicine ou son sel pharmaceutiquement acceptable ;
8-fluoro-carminomycine ou son sel pharmaceutiquement acceptable ;
8-fluoro-14-hydroxy-carminomycine ou son sel pharmaceutiquement acceptable ;
8-fluoro-4′-épi-carminomycine ou son sel pharmaceutiquement acceptable ;
8-fluoro-14-hydroxy-4′-épi-carminomycine ou son sel pharmaceutiquement acceptable ;
8-fluoro-4′-déoxy-carminomycine ou son sel pharmaceutiquement acceptable ;
8-fluoro-14-hydroxy-4′-déoxy-carminomycine ou son sel pharmaceutiquement acceptable ;
8-fluoro-daunorubicine ou son sel pharmaceutiquement acceptable ;
8-fluoro-doxorubicine ou son sel pharmaceutiquement acceptable ;
8-fluoro-4′-épi-daunorubicine ou son sel pharmaceutiquement acceptable ;
8-fluoro-4′-épi-doxorubicine ou son sel pharmaceutiquement acceptable ;
8-fluoro-4′-déoxy-daunorubicine ou son sel pharmaceutiquement acceptable ;
8-fluoro-4′-déoxy-doxorubicine ou son sel pharmaceutiquement acceptable ;
10-fluoro-4-déméthoxydaunorubicine ou son sel pharmaceutiquement acceptable ;
10-fluoro-4-déméthoxydoxorubicine ou son sel pharmaceutiquement acceptable ;
10-fluoro-4-déméthoxy-4′-épi-daunorubicine ou son sel pharmaceutiquement acceptable ;
10-fluoro-4-déméthoxy-4′-épi-doxorubicine ou son sel pharmaceutiquement acceptable ;
10-fluoro-4-déméthoxy-4′-déoxy-daunorubicine ou son sel pharmaceutiquement acceptable ;
10-fluoro-4-déméthoxy-4′-déoxy-doxorubicine ou son sel pharmaceutiquement acceptable ;
10-fluoro-carminomycine ou son sel pharmaceutiquement acceptable ;
10-fluoro-14-hydroxy-carminomycine ou son sel pharmaceutiquement acceptable ;
10-fluoro-4′-épi-carminomycine ou son sel pharmaceutiquement acceptable ;
10-fluoro-14-hydroxy-4′-épi-carminomycine ou son sel pharmaceutiquement acceptable ;
10-fluoro-4′-déoxy-carminomycine ou son sel pharmaceutiquement acceptable ;
10-fluoro-14-hydroxy-4′-déoxy-carminomycine ou son sel pharmaceutiquement acceptable ;
10-fluoro-daunorubicine ou son sel pharmaceutiquement acceptable ;
10-fluoro-doxorubicine ou son sel pharmaceutiquement acceptable ;
10-fluoro-4′-épi-daunorubicine ou son sel pharmaceutiquement acceptable ;
10-fluoro-4′-épi-doxorubicine ou son sel pharmaceutiquement acceptable ;
10-fluoro-4′-déoxy-daunorubicine ou son sel pharmaceutiquement acceptable ;
10-fluoro-4′-déoxy-doxorubicine ou son sel pharmaceutiquement acceptable.

8. Naphtacènedione de formule : 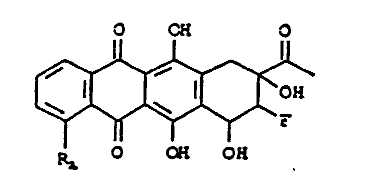 dans laquelle R₂ est un atome hydrogène ou un groupe hydroxyle ou un groupe alcoxy.

9. Naphtacènedione de formule : 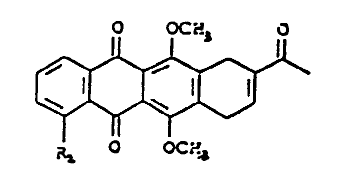 dans laquelle R₂ est un atome hydrogène ou un groupe hydroxyle ou un groupe alcoxy.

10. Naphtacènedione de formule : 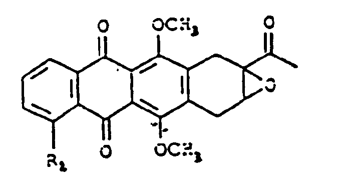 dans laquelle R₂ est un atome hydrogène ou un groupe hydroxyle ou un groupe alcoxy.

11. Naphtacènedione de formule : 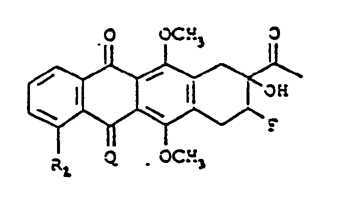 dans laquelle R₂ est un atome hydrogène ou un groupe hydroxyle ou un groupe alcoxy.

12. Naphtacènedione de formule : 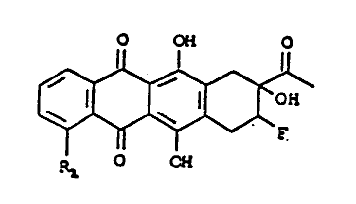 dans laquelle R₂ est un atome hydrogène ou un groupe hydroxyle ou un groupe alcoxy.

13. Naphtacènedione de formule : 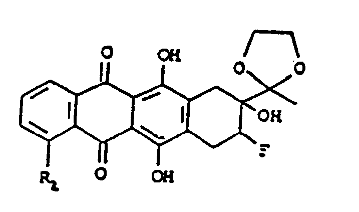 dans laquelle R₂ est un atome hydrogène ou un groupe hydroxyle ou un groupe alcoxy.

14. Naphtacènedione de formule : 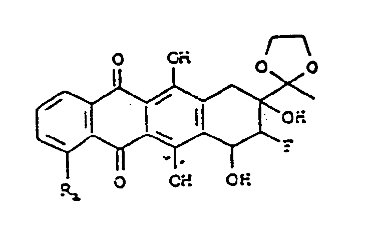 dans laquelle R₂ est un atome hydrogène ou un groupe hydroxyle ou un groupe alcoxy.

15. Utilisation d'un ou plusieurs des composés selon la revendication 3 ou 5 ou ses sels pharmaceutiquement acceptables en combinaison avec un vecteur ou diluant acceptable pharmaceutiquement pour la production d'une composition pharmaceutique utile comme agent chimiothérapeutique tout particulièrement vis-à-vis de l'activité antitumorale et antivirale.

16. Procédé pour la préparation des composés selon la revendication 1, lorsque R = H et R₁ = F, ce procédé assurant :
i) la cyclisation de la 1,4-diméthoxy-2,3-bis(bromométhyl)-anthraquinone judicieusement substituée par 3-butin-2-one avec ou sans présence d'un catalyseur approprié ;
ii) l'époxydation avec un composé peroxy ;
iii) l'ouverture du cycle oxyrane avec un fluor nucléophile;
iv) la déméthylation des hydroxyles phénoliques et protection de la chaîne acétyle ;
v) la bromination de la naphtacènedione en position 4 avec du brome ou un autre agent de bromation approprié tel que N-bromo-succinimide ou 1,3-dibromo-5,5-diméthyldantoïne ou poly(4-vinylpyridin bromohydrate perbromure) supporté par un polymère ; substitution ultérieure avec un hydroxyle ; et pour finir enlèvement de la protection du groupe acétyle.

17. Procédé selon la revendication 16, pour l'obtention des composés selon la revendication 1 avec X = OH dans lequel un atome bromure est introduit sur le groupe acétyle de la 7-substituée fluoro-2β-acétyl-1,2-époxy-4α,5,12-trihydroxy-1,2,3,4-tétrahydro-6,11-naphtacènedione, puis substitution par un hydroxyle soit au moyen de l'hydrolyse directe du dérivé brome soit par hydrolyse d'un ester approprié tel qu'un formate obtenu à partir de celui-ci par réaction d'échange ; ou encore l'hydroxyle est maintenu dans une forme judicieusement protégée.

18. Procédé pour la préparation des composés selon la revendication 1, lorsque R = F et R₁ = H, ce procédé impliquant l'ouverture du cycle oxyrane de la 7-substituée-2β-acétyl-1,2-époxy-4α,5,12-trihydroxy-1,2,3,4-tétrahydro-6,11-naphtacènedione avec un fluor nucléophile.

19. Procédé pour la préparation des composés selon la revendication 3 ou 5, ce procédé assurant la glycosidation des composés obtenus par le procédé de la revendication 16 ou 17 ou 18 avec un dérivé glucidique judicieusement protégé en présence d'agents de condensation tels que du triflate d'argent ou du perchlorate d'argent, de l'oxyde de mercure et des mélanges de bromure, du triméthylsilyltriflate, ou des acides tels que l'acide p-toluènesulfonique ou des acides Lewis tels que un trihalogénure de bore, un tétrachlorure d'étain ou des composés chimiques avec des propriétés équivalentes avec ou sans la présence d'un agent déshydratant approprié.

20. Procédé selon la revendication 19, dans lequel l'enlèvement de la protection du composé obtenu s'effectue avec des agents basiques si le groupe protecteur est trifluoroacétyle ou avec des complexes nickel ou palladium si le groupe protecteur est allyloxycarbonyle.

21. Procédé pour la préparation des composés selon la revendication 3, en partant de 7-substituées 4-glycosilées 2β-acétyl-2α,4α,5,12-tétrahydroxy-1,2,3,4-tétrahydro-6,11-naphtacènediones par l'introduction d'un atome de brome sur le groupe acétyle en position 2 et substitution par un hydroxyle soit à l'aide de l'hydrolyse directe du dérivé brome soit à l'aide de l'hydrolyse d'un ester approprié tel qu'un formate obtenu par celui-ci au moyen d'une réaction d'échange.
